(19) Europäisches Patentamt
European Patent Office
Office européen des brevets

(11) **EP 4 139 668 B1**

(12) **FASCICULE DE BREVET EUROPEEN**

(45) Date de publication et mention
de la délivrance du brevet:
**31.07.2024 Bulletin 2024/31**

(21) Numéro de dépôt: **21731222.2**

(22) Date de dépôt: **23.04.2021**

(51) Classification Internationale des Brevets (IPC):
**G01N 23/222** (2006.01)   **G01N 1/40** (2006.01)
**G01N 23/223** (2006.01)   **G01N 33/24** (2006.01)

(52) Classification Coopérative des Brevets (CPC):
**G01N 23/222; G01N 1/4055; G01N 23/223;
G01N 33/24**

(86) Numéro de dépôt international:
**PCT/FR2021/050707**

(87) Numéro de publication internationale:
**WO 2021/214417 (28.10.2021 Gazette 2021/43)**

(54) **METHODE DE DETERMINATION DE LA VALORISABILITE DE MATERIAUX EXCAVES**

**VERFAHREN ZUR BESTIMMUNG DER NUTZBARKEIT VON AUSHUBMATERIAL**

**METHOD FOR DETERMINING THE USABILITY OF EXCAVATED MATERIAL**

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **24.04.2020 FR 2004118**

(43) Date de publication de la demande:
**01.03.2023 Bulletin 2023/09**

(73) Titulaire: **Eiffage GC Infra Linéaires
78140 Vélizy-Villacoublay (FR)**

(72) Inventeurs:
• **DOOM, Florian
78110 LE VESINET (FR)**
• **BOULANGE, Laurence
38530 Chapareillan (FR)**

(74) Mandataire: **Plasseraud IP
66, rue de la Chaussée d'Antin
75440 Paris Cedex 09 (FR)**

(56) Documents cités:
**FR-A1- 3 062 917    JP-A- 2011 247 694
US-A- 3 262 310**

• **MATSUMOTO SHINJI ET AL: "Characterization of
Mine Waste and Acid Mine Drainage Prediction
by Simple Testing Methods in Terms of the
Effects of Sulfate-Sulfur and Carbonate
Minerals", MINERALS, vol. 8, no. 9, 1 September
2018 (2018-09-01), pages 403, XP055776845,
ISSN: 2075-163X, DOI: 10.3390/min8090403**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

### Domaine technique

La présente divulgation relève du domaine de la revalorisation des matériaux excavés en tant que matériau de construction, en particulier en tant que remblai ou granulat.

### Contexte de l'invention

Lors de grands travaux en génie civil, notamment lors de creusement de tunnels, une quantité très importante de matériaux excavés est générée en très peu de temps, ce qui nécessite une forte emprise sur les chantiers. Par exemple, un tunnelier utilisé pour la construction d'une ligne de métro extrait environ 800 tonnes par jour de matériaux excavés.

Classiquement les matériaux excavés sont stockés dans des installations de stockage de déchets ayant une empreinte au sol très importante. Pour éviter ce problème, une solution consiste à revaloriser ces matériaux excavés en nouveaux matériaux de construction.

Cependant les matériaux excavés contiennent de nombreux composés chimiques inorganiques différents qui, dans le temps, peuvent dégrader les propriétés de ces nouveaux matériaux de construction et altérer les ouvrages dans lesquels ils sont mis en oeuvre.

C'est pourquoi il est nécessaire de déterminer la concentration massique en composés chimiques inorganiques dans un matériau excavé avant de décider de le valoriser ou non.

La procédure pour déterminer la concentration massique des composés chimiques inorganiques est fixée par des normes nationales. En France, il s'agit des normes françaises NF EN 12457-2 et NF EN 16192. Selon cette norme, la quantité de composés chimiques inorganiques dans un matériau excavé est déterminée par une analyse physicochimique de la composition de l'eau résultante de la lixiviation simulée dudit matériau excavé. Selon cette norme française, une lixiviation (traitement par l'eau bien connu qui entraîne la dissolution des espèces solubles) du matériau excavé est simulée pendant 24 heures à température ambiante (20°C $\pm$ 5°C). L'eau est ensuite filtrée pour être analysée. L'opération de filtration peut être fastidieuse du fait de particules argileuses qui colmatent les filtres ce qui augmente le temps de rendu d'analyse. Au final, cette opération prend au minimum 48 heures. Il faut également ajouter à cette durée le délai de transport vers un laboratoire externe au site d'extraction des matériaux excavés et le délai de traitement pour une durée totale générale de sept jours. Durant cette longue période de détermination, il est nécessaire de stocker les matériaux excavés extraits sur les chantiers qui se trouvent très souvent implantés dans des zones géographiques à très fortes contraintes (zone urbaine, vallée montagneuse). Une autre procédure connue est décrite par MATSUMOTO SHINJI ET AL ("Characterization of Mine Waste and Acid Mine Drainage Prédiction by Simple Testing Methods in Terms of the Effects of Sulfate-Sulfur and Carbonate Minerals",MINERALS, vol. 8, no. 9, 1 septembre 2018). L'article décrit un procédé de détermination du caractère d'un matériau excavé en déterminant la concentration totale en soufre par XRF et la fraction lixiviables en sulfate par extraction solide/liquide.

Il existe donc un besoin d'un procédé pour déterminer rapidement si, et éventuellement comment, des matériaux excavés peuvent être valorisés pour optimiser les emprises de chantier, pour éviter les zones de stockage sur le chantier, pour permettre la revalorisation sûre d'une quantité importante de matériaux excavés.

De façon surprenante, la Demanderesse a trouvé un procédé de détermination du caractère valorisable d'un matériau excavé permettant d'écarter ces inconvénients.

### Résumé

Selon un premier aspect, la présente invention porte sur un procédé de détermination du caractère valorisable d'un matériau excavé comprenant les étapes suivantes :

a) analyse d'activation par neutrons gamma rapides, analyse par spectroscopie par fluorescence aux rayons X ou leur combinaison d'un premier échantillon du matériau excavé pour déterminer la concentration massique en soufre ($S_{total}$) et la concentration massique en chlore ($Cl_{total}$) du matériau excavé,

b) extraction solide/liquide à une température $T_{extraction}$ de 65°C à 200°C, en particulier de 70°C à 150°C, plus particulièrement de 85°C à 115°C d'un second échantillon du matériau excavé avec un solvant liquide pour obtenir un mélange comprenant un lixiviat et une fraction solide,

c) analyse chimique, analyse physique ou leur combinaison dudit lixiviat pour déterminer la concentration massique en sulfates lixiviables ($SO_4^{2-}{}_{soluble}$) et la concentration massique en chlorures lixiviables ($Cl^-{}_{soluble}$) du matériau excavé.

A partir des résultats obtenus par le procédé de détermination selon l'invention, il peut être possible de :

- déterminer un indice de réactivité (IR) du matériau excavé et comparer cet indice de réactivité (IR) avec une première valeur maximale,
- comparer la concentration massique en sulfates lixiviables ($SO_4^{2-}{}_{soluble}$) du matériau excavé avec une seconde valeur maximale, et
- déterminer le caractère valorisable du matériau excavé selon les critères suivants :
  - si l'indice de réactivité (IR) est inférieur ou égal à la première valeur maximale et si la concentration massique en sulfates lixiviables ($SO_4^{2-}$ soluble) est inférieure ou égale à la seconde valeur maximale alors le matériau excavé est considéré valorisable,

- si l'indice de réactivité (IR) est supérieur à la première valeur maximale et/ou si la concentration massique en sulfates lixiviables ($SO_4^{2-}$ soluble) est supérieure à la seconde valeur maximale alors le matériau excavé est considéré comme non valorisable,

l'indice de réactivité (IR) étant déterminé selon l'équation suivante :

$$IR = 100 * \frac{0{,}33 * SO_{4\,soluble}^{2-} + Cl_{soluble}^{-}}{S_{total} + Cl_{total}}$$

Sans vouloir être liés par aucune théorie; les inventeurs sont d'avis que l'indice de réactivité et la concentration massique en sulfates lixiviables d'un matériau excavé permettent de déterminer l'évolution des propriétés d'un matériau de construction comprenant ce matériau excavé. En effet les composés soufrés les composés chlorés sont les composés chimiques altérant le plus les matériaux de construction. Par exemples les sulfates sont les composés les plus instables et peuvent engendrer une rupture de béton par gonflement. Les composés chlorés, quant à eux, engendrent des réactions de corrosion des aciers utilisés dans des bétons armés.

De façon avantageuse, le procédé de détermination selon l'invention permet de déterminer de façon fiable et en accord avec les recommandations actuelles si un matériau excavé peut être valorisé.

De plus, cette détermination est très rapide car les analyses mises en oeuvre dans les étapes a) et c) permettent de déterminer la concentration massique en soufre, en chlore, sulfates lixiviables et en chlorures lixiviables du matériau excavé de réactivité en quelques dizaines de minutes. De plus la durée de l'étape b) d'extraction, qui correspond à la durée durant laquelle la température $T_{extraction}$ est maintenue dans la plage indiquée ci-dessus, peut être inférieure à 60 secondes, voire même inférieure à 30 secondes.

Selon un second aspect, la présente invention porte également sur un procédé de sélection d'une filière de valorisation d'un matériau excavé mettant en oeuvre le procédé de détermination selon l'invention et comprenant en outre l'étape suivante :
d) mesure de la résistance en compression d'un troisième échantillon de matériau excavé.

Les inventeurs ont également trouvé que l'indice de réactivité (IR), la concentration massique en sulfates lixiviables et la résistance en compression d'un matériau excavé permettent de conclure de façon fiable, rapidement et en accord avec les recommandations actuelles en quel matériau de construction ce matériau excavé peut être valorisé, par exemple en remblai ou en granulat.

En effet, à partir des résultats obtenus par le procédé de sélection de l'invention, il peut être possible de :

- déterminer l'indice de réactivité (IR) du matériau excavé et comparer cet indice de réactivité (IR) avec une première valeur maximale,
- comparer la concentration massique en sulfates lixiviables ($SO_4^{2-}$ soluble) du matériau excavé avec une seconde valeur maximale,
- comparer la résistance en compression du matériaux excavé avec une valeur seuil, et
- sélectionner la filière de valorisation du matériau excavé selon les critères suivants :
- le matériau excavé peut être valorisé en tant que remblai si sa résistance en compression est inférieure à la valeur seuil, et

  - soit son indice de réactivité (IR) est compris entre la première valeur minimale et la première valeur maximale et sa concentration massique en sulfates lixiviables ($SO_4^{2-}$ soluble) est inférieure ou égale à la seconde valeur maximale,
  - soit sa concentration massique en sulfates lixiviables ($SO_4^{2-}$ soluble) est comprise entre la seconde valeur minimale et la seconde valeur maximale et son indice de réactivité (IR) est inférieur ou égal à la première valeur maximale ;

- le matériau excavé peut être valorisé en tant que granulat si :

  sa résistance en compression est supérieure à la valeur seuil, et
  son indice de réactivité (IR) est inférieur ou égal à la première valeur minimale et sa concentration massique en sulfates lixiviables ($SO_4^{2-}$ soluble) est inférieure ou égale à la seconde valeur maximale.

Selon un autre aspect, il est proposé un procédé de production d'un matériau de construction comprenant une ou des étapes de mise en forme d'un matériau excavé dont un ou des échantillons ont subi les étapes a) à c) du procédé de détermination selon l'invention et l'étape d) du procédé de sélection selon l'invention.

Selon un autre aspect, il est proposé un matériau de construction comprenant un matériau excavé valorisable présentant un indice de réactivité (IR) inférieur ou égal à une première valeur maximale et une concentration massique en sulfates lixiviables inférieure ou égale à une seconde valeur maximale,
l'indice de réactivité (IR) étant déterminé selon l'équation suivante :

$$IR = 100 * \frac{0{,}33 * SO_{4\ soluble}^{2-} + Cl_{soluble}^{-}}{S_{total} + Cl_{total}}$$

avec $SO_{4\ soluble}^{2-}$ la concentration massique en sulfates lixiviables du matériau excavé, $Cl_{soluble}^{-}$ la concentration massique en chlorures lixiviables du matériau excavé, $S_{total}$ la concentration massique en soufre total du matériau excavé et $Cl_{total}$ la concentration massique en chlore total du matériau excavé.

Un exemple, qui ne forme pas partie de l'invention, concerne un laboratoire mobile comprenant des équipements de laboratoire adaptés à la mise en oeuvre d'au moins une des étapes a), b) et c) du procédé de détermination selon l'invention et/ou des équipements de laboratoire adaptés à la mise en oeuvre de l'étape d) du procédé de sélection selon l'invention.

**Exposé de l'invention**

Selon un premier aspect, la présente invention porte sur un procédé de détermination du caractère valorisable d'un matériau excavé comprenant les étapes suivantes :

> a) analyse d'activation par neutrons gamma rapides, analyse par spectroscopie par fluorescence aux rayons X ou leur combinaison d'un premier échantillon du matériau excavé pour déterminer la concentration massique en soufre ($S_{total}$) et la concentration massique en chlore ($Cl_{total}$) du matériau excavé,
> b) extraction solide/liquide à une température $T_{extraction}$ de 65°C à 200°C, en particulier de 70°C à 150°C, plus particulièrement de 85°C à 115°C d'un second échantillon du matériau excavé avec un solvant liquide pour obtenir un mélange comprenant un lixiviat et une fraction solide,
> c) analyse chimique, analyse physique ou leur combinaison dudit lixiviat pour déterminer la concentration massique en sulfates lixiviables ($SO_{4\ soluble}^{2-}$) et la concentration massique en chlorures lixiviables ($Cl_{soluble}^{-}$) du matériau excavé.

Comme expliqué ci-dessus, le procédé de détermination selon l'invention permet avantageusement de déterminer en quelques minutes, de façon fiable et en accord avec les recommandations actuelles si un matériau excavé peut être valorisé en matériau de construction.

Selon un mode de réalisation, le procédé de détermination peut comprendre les étapes suivantes :

- détermination d'un indice de réactivité (IR) du matériau excavé et comparaison de cet indice de réactivité (IR) avec une première valeur maximale,
- comparaison de la concentration massique en sulfates lixiviables ($SO_{4\ soluble}^{2-}$) du matériau excavé avec une seconde valeur maximale, et

- détermination du caractère valorisable du matériau excavé selon les critères suivants :

- si l'indice de réactivité (IR) est inférieur ou égal à la première valeur maximale et si la concentration massique en sulfates lixiviables ($SO_{4\ soluble}^{2-}$) est inférieure ou égale à la seconde valeur maximale alors le matériau excavé est considéré valorisable,
- si l'indice de réactivité (IR) est supérieur à la première valeur maximale et/ou si la concentration massique en sulfates lixiviables ($SO_{4\ soluble}^{2-}$) est supérieure à la seconde valeur maximale alors le matériau excavé est considéré comme non valorisable,

l'indice de réactivité étant déterminé selon l'équation suivante :

$$IR = 100 * \frac{0{,}33 * SO_{4\ soluble}^{2-} + Cl_{soluble}^{-}}{S_{total} + Cl_{total}}$$

Au sens de la présente demande, "matériau excavé" (aussi appelé "matériau d'excavation") désigne tout matériau issu de travaux de génie civil ou de construction que ce soit à la surface de la Terre, par exemple lors de fouilles ou de création de fondation, ou dans le sous-sol, par exemple lors du creusement de tunnels, cavernes et galeries. Le matériau excavé comprend alors des :

- roches meubles tels que graviers, sables, limons, argiles ou leurs mélanges;
- rochers concassés;

- matériaux provenant de constructions antérieures ou de sites pollués tels que des décharges; ou

  - boues.

Par exemple, le matériau excavé est le matériau extrait par un tunnelier utilisé pour la construction d'une ligne de métro, d'une ligne de train, d'une route.

Le matériau excavé peut présenter un taux de matière sèche, ou siccité, de 70% à 100%, en particulier de 75% à 90%, tout particulièrement de 78% à 82%.

Au sens de la présente demande, le "taux de matière sèche" est le rapport entre la masse sèche de matériau excavé et la masse de matériau excavé avant séchage, la masse sèche du matériau excavé étant mesurée après séchage d'environ 30 grammes de matériau excavé pendant 30 minutes à 130°C.

Au sens de la présente demande, "analyse d'activation par neutrons gamma rapides" (*Prompt-Gamma Neutron Activation Analysis* ou PGNAA selon la terminologie anglaise) désigne une technique d'analyse des rayons gamma spécifiques qui ont été émis pendant la désintégration après que ces derniers s'irradient dans un réacteur nucléaire. Ces rayons gamma sont spécifiques à chaque élément chimique. Les rayons gammas vont donc permettre l'identification des éléments chimiques présents dans l'échantillon et leur taux de comptage est proportionnel à la concentration massique en éléments chimiques présents dans l'échantillon.

Au sens de la présente demande, "spectroscopie par fluorescence aux rayons X" désigne une technique d'analyse chimique utilisant une propriété physique de la matière, la fluorescence aux rayons X. Les atomes possèdent tous un nombre d'électrons définis, et chacun de ces électrons a une énergie propre définie par son niveau atomique. Les niveaux atomiques correspondent à une énergie et peuvent contenir un nombre d'électrons précis. Plus un niveau d'énergie est haut en énergie, plus il y a de place mais moins les électrons auront tendance à le remplir. Un électron peut être excité par un photon et changer de niveau d'énergie, passant à un niveau d'énergie plus élevée. La place laissée dans le niveau d'énergie par l'électron est alors comblée par un électron d'un autre niveau d'énergie. La transition de niveau d'énergie libère de l'énergie, sous différentes formes. Le phénomène qui nous intéresse pour la fluorescence aux rayons X est l'émission d'un photon de fluorescence dans le domaine des rayons X. L'énergie de chaque photon émis de la sorte est précisément connue pour chaque élément et les différentes transitions possibles pour un élément sont décrites. Il existe ainsi des tables donnant pour chaque élément toutes les énergies d'émission possible et l'énergie minimale pour exciter l'atome. Le spectre obtenu par la spectroscopie par fluorescence aux rayons X est caractéristique de l'échantillon analysé.

De façon avantageuse, l'analyse d'activation par neutrons gamma rapides et l'analyse par spectroscopie par fluorescence aux rayons X permettent de déterminer la composition élémentaire d'un échantillon de matériau excavé et de déterminer la concentration massique de chaque élément du matériau excavé, en particulier la concentration massique en chlore ($Cl_{total}$) et la concentration massique en soufre ($S_{total}$) du matériau excavé. De plus cette technique est particulièrement rapide car la durée des mesures est de quelques dizaines de minutes et elle est non destructive.

Selon un mode de réalisation préférée, l'étape a) d'analyse est réalisée par analyse par spectroscopie par fluorescence aux rayons X. En effet la spectroscopie par fluorescence aux rayons X est plus simple à mettre en oeuvre que l'analyse d'activation par neutrons gamma rapides car elle ne requière pas de source radioactive.

Au sens de la présente demande, "concentration massique en chlore ($Cl_{total}$)" désigne la concentration massique de tous les composés comprenant du chlore présents dans le matériau excavé.

Au sens de la présente demande, "concentration massique en soufre ($S_{total}$)" désigne la concentration massique de tous les composés comprenant du soufre présents dans le matériau excavé.

Au sens de la présente demande, "lixiviat" désigne la fraction liquide résultant de l'étape b) d'extraction solide/liquide.

Au sens de la présente demande, "analyse chimique" désigne une analyse dont le principe est lié à une réaction chimique.

Au sens de la présente demande, "analyse physique" désigne une analyse reposant sur un principe physique.

De façon avantageuse, l'analyse chimique et l'analyse physique permettent de déterminer rapidement les caractéristiques du lixiviat, en particulier sa concentration en ions chlorures et sa concentration en ions sulfates appelées respectivement, concentration massique en chlorures lixiviables ($Cl^-_{lixiviables}$) du matériau excavé et concentration massique en sulfates lixiviables ($SO_4^{2-}_{lixiviables}$) du matériau excavé.

Au sens de la présente demande, "première valeur maximale" désigne un nombre décimal positif reflétant la concentration massique en composés soufrés et/ou la concentration massique en composés chlorées d'un matériau excavé au-dessus de laquelle les propriétés d'un matériau de construction comprenant ledit matériau excavé vont se dégrader dans le temps. Typiquement, la première valeur maximale est comprise entre 27 et 33, en particulier entre 28,5 et 31,5, tout particulièrement de 30.

Au sens de la présente demande, "seconde valeur maximale" désigne la concentration en sulfates lixiviables dans le matériau excavé, en ppm, au-dessus de laquelle les propriétés d'un matériau de construction comprenant ledit matériau excavé vont se dégrader dans le temps. Typiquement la seconde valeur maximale est comprise entre 2700 ppm et 4400 ppm, en particulier entre 2850 ppm et 4150ppm, tout particulièrement entre 3000 ppm et 4000 ppm.

De façon avantageuse, la conclusion du caractère valorisable d'un matériau excavé est en accord avec les recommandations actuelles lorsque la première valeur maximale et la seconde valeur maximale sont dans les plages décrites ci-dessus.

Selon un mode de réalisation, l'étape a) d'analyse peut être réalisée directement sur le matériau excavé, le premier échantillon étant alors le matériau excavé, ou sur un prélèvement du matériau excavé.

Selon un mode de réalisation, l'analyse au cours de l'étape a) par spectroscopie par fluorescence aux rayons X est réalisée avec une énergie variant de 0 à 100 keV, en particulier de 0 à 50 keV,

Selon un mode de réalisation particulier, l'analyse au cours de l'étape a) par spectroscopie par fluorescence aux rayons X est réalisée sur une ou plusieurs plages d'énergie, chaque plage d'énergie allant de -5 keV, en particulier de -3 keV, tout particulièrement de -2 keV d'une énergie centrale à +5 keV, en particulier à +3 keV, tout particulièrement à +2 keV de l'énergie centrale, l'énergie centrale étant l'énergie d'une raie caractéristique du chlore et du soufre.

De façon avantageuse, réaliser l'analyse par spectroscopie par fluorescence aux rayons X sur différentes plages d'énergie permet d'augmenter le signal reçu et donc d'augmenter le rapport signal/bruit. En conséquence, le chlore et le soufre contenus dans l'échantillon analysé sont plus facilement détectés et plus facilement dosés.

Par exemple, l'énergie centrale du chlore est 2,62 keV, 2,82 keV ou les deux. L'énergie centrale du soufre, quant à elle, est de 2,31 keV, 2,46 keV ou les deux.

Selon un mode de réalisation, le temps total d'acquisition lors de l'analyse par spectroscopie par fluorescence aux rayons X au cours de l'étape a) est de 1 min à 120 min, en particulier de 5 min à 60 min, tout particulièrement est de 10 min.

De façon avantageuse, un temps total d'acquisition dans ces plages de valeurs est très court et permet de recevoir un signal de qualité et donc d'améliorer la fiabilité du dosage des éléments polluants inorganiques contenus dans l'échantillon analysé.

Selon un mode de réalisation, lors de l'analyse par spectroscopie par fluorescence aux rayons X au cours de l'étape a), les électrons sont accélérés par une tension d'accélération de 5 kV à 200 kV, en particulier de 25 kV à 100 kV, plus particulier une tension d'accélération de 50 kV.

De façon avantageuse, une valeur de tension d'accélération dans ces plages permet d'exciter à la fois le chlore et le soufre. Cela permet donc de déterminer la concentration massique de chlore et en soufre contenus dans l'échantillon analysé.

Typiquement l'étape b) d'extraction est réalisée avec un solvant liquide choisi parmi l'eau, une solution aqueuse, un solvant organique, un solvant inorganique et leurs mélanges.

Typiquement le solvant organique est un solvant organique volatile, en particulier un solvant organique volatile choisi parmi le méthanol, l'acétone, l'hexane, l'acétonitrile, l'éthanol, un éther, le diméthylsulfoxyde, le 2-hexanone et leurs mélanges.

Selon un mode de réalisation particulier le solvant est l'eau.

Typiquement, le ratio entre le volume de solvant et la masse sèche du second échantillon du matériau excavé lors de l'étape b) d'extraction est de 5 ml/g à 20 ml/g, en particulier de 7 ml/g à 15 ml/g, tout particulièrement de 9,5 ml/g à 10,5 ml/g.

Selon un premier mode de réalisation, le second échantillon du matériau excavé et/ou le solvant sont chauffés à la température $T_{extraction}$, puis ils sont mis en contact pour mettre en oeuvre l'étape b) d'extraction.

Selon un deuxième mode de réalisation, le second échantillon du matériau excavé est mis en contact avec le solvant pour obtenir un mélange, puis ce mélange est chauffé jusqu'à la température $T_{extraction}$ pour mettre en oeuvre l'étape b) d'extraction.

Selon un troisième mode de réalisation, le second échantillon du matériau excavé et/ou le solvant sont chauffés à la température T1, inférieure à la température $T_{extraction}$, puis ils sont mis en contact pour obtenir un mélange, ce mélange est ensuite chauffé jusqu'à la température $T_{extraction}$ de sorte à réaliser l'étape b) d'extraction.

Typiquement la température T1 est de 40°C à 80°C, en particulier de 45°C à 70°C, tout particulièrement de 50°C à 65°C.

Typiquement le solvant et le second échantillon du matériau excavé sont mis en contact de sorte à améliorer la cinétique d'homogénéisation du mélange échantillon/solvant liquide et donc diminuer la durée de l'étape b) d'extraction.

L'étape b) d'extraction étant réalisée à $T_{extraction}$, l'étape b) peut être réalisée à une pression $P_{extraction}$ afin de garder le solvant sous forme liquide. Typiquement $P_{extraction}$ peut être de 1 bar à 10 bar, en particulier de 1,2 bar à 5 bar, tout particulièrement de 1,6 bar à 2,5 bar.

De façon avantageuse, la réalisation de l'étape b) d'extraction sous la pression $P_{extraction}$ permet au solvant liquide de s'introduire plus rapidement dans l'échantillon du matériau excavé qu'à 1 bar. De ce fait la durée de l'étape b) d'extraction est diminuée.

Selon un mode de réalisation particulier, l'étape b) d'extraction est réalisée dans un réacteur comprenant :

- une chambre d'extraction adaptée pour recevoir le second échantillon du matériau excavé, et
- deux ports d'entrée de liquide et un port de sortie de liquide,

dans lequel
les deux ports d'entrée de liquide sont fluidiquement connectés à la chambre d'extraction et positionnés de part et d'autre de la chambre d'extraction, et le port de sortie de liquide est fluidiquement connecté à la chambre d'extraction.

Au sens de la présente demande, on entend par "port d'entrée de liquide" tout élément adapté à introduire le solvant dans la chambre d'extraction du réacteur.

Au sens de la présente demande, on entend par "port de sortie de liquide" tout élément adapté à extraire la fraction liquide de la chambre d'extraction du réacteur.

La position des deux ports d'entrée de liquide, de part et d'autre de la chambre d'extraction, permet d'améliorer l'homogénéité du mélange échantillon/solvant. De façon avantageuse, ceci permet de faciliter l'extraction solide/liquide et donc de réduire la durée de l'étape b) d'extraction.

Le lixiviat obtenu lors de l'étape b) d'extraction comprend le sulfates lixiviables et le chlorures lixiviables compris dans le second échantillon du matériau excavé. L'analyse lors de l'étape c) du lixiviat permet de déterminer la concentration massique des sulfates lixiviables et des chlorures lixiviables du matériau excavé.

L'analyse chimique, mise en oeuvre dans étape c), peut être un dosage conductimétrique, un dosage par chromatographie ionique en phase liquide, un dosage colorimétrique, un dosage potentiométrique, un dosage pH-métrique, ou leurs combinaisons, en particulier un dosage conductimétrique, un dosage potentiométrique ou leur combinaison.

L'analyse physique, mise en oeuvre dans étape c), peut être une spectrométrie d'absorption, une spectrométrie d'émission atomique avec plasma à couplage inductif (ICP-AES), une spectrométrie de masse avec plasma à couplage inductif (ICP-MS), une spectrométrie d'ionisation de flamme (FID), une spectrométrie d'émission de flamme, une spectrophotométrie UV, ou leurs combinaisons, en particulier une spectrométrie de masse avec plasma à couplage inductif (ICP-MS).

Ces méthodes d'analyses sont connues de l'homme du métier qui saura les adapter pour déterminer la concentration massique en chlorures lixiviables et en sulfates lixiviables du matériau excavé.

Selon un mode de réalisation particulier, la concentration massique en chlorures lixiviables peut être déterminée par dosage potentiométrique et la concentration massique en sulfates lixiviables peut être déterminée par dosage conductimétrique.

Le dosage potentiométrique met en oeuvre une électrode adaptée pour détecter les ions chlorures. Le potentiel mesuré entre cette électrode et une électrode référence est directement lié à la concentration en chlorures. L'utilisation d'un système étalonné permet alors de déterminer la concentration en chlorures lixiviables.

Les cations de la solution titrante peuvent être le baryum, le calcium, le cuivre, le fer, le manganèse, le zinc ou leur mélange, en particulier le baryum, le cuivre, le fer ou leur mélange, tout particulièrement le baryum.

La concentration en cation de la solution titrant peut, par exemple, être compris entre 0,01 mol/L et 1 mol/L, en particulier entre 0,25 mol/L et 0,5 mol/L, tout particulièrement entre 0,04 mol/l et 0,06 mol/L.

Le dosage conductimétrique met en oeuvre une solution comprenant les ions sulfate dans laquelle est ajoutée au goutte à goutte une solution titrante comprenant des cations qui réagissent avec les ions sulfates. Le suivi de la conductivité de la solution comprenant les ions sulfates permet de détecter la consommation des ions sulfates par les cations de la solution titrante. La conductivité augmente lentement avec l'ajout de la solution titrante tant qu'il y a consommation des ions sulfates puis augmente fortement lorsque les ions sulfates ont été entièrement consommés. L'intersection des pentes donne le volume équivalent qui permet de déterminer la concentration en sulfates lixiviables.

Les cations de la solution titrante peuvent être le baryum, le calcium, le cuivre, le fer, le manganèse, le zinc ou leur mélange, en particulier le baryum, le cuivre, le fer ou leur mélange, tout particulièrement le baryum.

La concentration en cation de la solution titrant peut, par exemple, être comprise entre 0,01 mol/L et 1 mol/L, en particulier entre 0,25 mol/L et 0,5 mol/L, tout particulièrement entre 0,04 mol/l et 0,06 mol/L.

Lorsque ces méthodes d'analyse sont mises en oeuvre dans l'étape c) d'analyse alors la durée de l'étape c) d'analyse est d'environ 60 minutes.

Selon un mode de réalisation, le procédé de détermination de l'invention peut comprendre, entre l'étape b) d'extraction et l'étape c) d'analyse, une étape de séparation du mélange comprenant le lixiviat et la fraction solide pour obtenir le lixiviat, cette étape de séparation étant réalisée à l'aide d'un filtre dont le matériau est choisi parmi une membrane de filtration, de la fibre de verre, de la cellulose, le PTFE, du nylon, du PMMA, du PE, des matériaux sulfonés, des matériaux acryliques, des matériaux fluorés, en particulier de la cellulose.

De façon avantageuse, cette étape de séparation permet de simplifier l'étape c) d'analyse et améliorer la qualité des résultats obtenus par cette étape c).

Au sens de la présente demande, on entend par "filtre" tout élément à travers lequel le lixiviat peut passer et retenir la fraction solide.

Le matériau du filtre peut être hydrophile ou hydrophobe. Selon un mode de réalisation particulier le matériau du filtre est hydrophile.

Le filtre peut présenter une porosité inférieure à 100 $\mu$m, en particulier inférieure à 75 $\mu$m, plus particulièrement inférieure à 55 $\mu$m. De façon avantageuse, une telle porosité favorise la séparation entre le lixiviat et la fraction solide.

Le ratio entre le volume du second échantillon du matériau excavé et la surface de filtration du filtre peut est inférieur à 10 cm, en particulier de 0.1 cm à 5 cm, tout particulièrement de 0,5 cm à 1 cm. De façon avantageuse, le filtre ne colmate pas lors de l'étape b) de filtration lorsque le ratio entre le volume de matériau excavé et la surface de filtration du filtre est compris dans les plages ci-dessus.

Par exemple l'étape de séparation peut être réalisée en appliquant une surpression au mélange comprenant le lixiviat et la fraction solide pour forcer ledit lixiviat à traverser le filtre, ou en appliquant une dépression au mélange comprenant le lixiviat et la fraction solide pour aspirer ledit lixiviat à travers le filtre, en particulier en appliquant une surpression.

L'homme du métier sait s'il doit appliquer une surpression ou une dépression au mélange comprenant le lixiviat et la fraction solide pour réaliser l'étape b) de filtration.

La surpression appliquée au mélange peut être supérieure à 1 bar, en particulier à 1,2 bar à 5 bar, tout particulièrement de 1,6 bar à 2,5 bar. La dépression appliquée au mélange peut être inférieure à 1 bar, en particulier de 0,2 bar à 0,5 bar.

Selon un mode de réalisation très particulier, l'étape de séparation est réalisée sous vide.

Lorsque l'étape b) d'extraction est réalisée dans le réacteur décrit ci-dessus, le filtre est positionné dans ledit réacteur de sorte que le port de sortie de liquide est fluidiquement connecté à la chambre d'extraction à travers le filtre.

Bien que la durée de l'étape de séparation dépende des paramètres décrits ci-dessus elle est typiquement inférieure à 10 minutes.

Pour réduire encore plus la durée du procédé de détermination de l'invention, il est possible de préparer les échantillons analysés. Ainsi, le procédé de détermination de l'invention peut comprendre une étape de préparation du premier échantillon et/ou du second échantillon avant l'étape a) d'analyse et l'étape b) d'extraction.

Le premier échantillon, le second échantillon, ou les deux peuvent subir, respectivement, avant l'étape a) et l'étape b) au moins une sous-étape choisie parmi :

- une sous-étape de prélèvement de l'échantillon ;
- une sous-étape de séchage de l'échantillon ;
- une sous-étape de broyage de l'échantillon ;
- une sous-étape de tamisage de l'échantillon, et
- une sous-étape de pastillage de l'échantillon.

Quelques dizaines de grammes d'échantillon suffisent pour la mise en oeuvre du procédé de détermination selon l'invention. La sous-étape de prélèvement de l'échantillon peut donc être réalisée par des méthodes classiques connues de l'homme du métier.

La sous-étape de broyage permet de diminuer la granulométrie de l'échantillon du matériau excavé à analyser et d'améliorer son homogénéité, ce qui permet, entre autres, de faciliter l'extraction solide/liquide et donc de réduire la durée de l'étape b). Typiquement, la granulométrie de l'échantillon après la sous-étape de broyage est inférieure à 5 mm, en particulier de 5 $\mu$m à 1000 $\mu$m, plus particulièrement de 20 $\mu$m à 500 $\mu$m.

Si nécessaire, la sous-étape de broyage peut être suivie par la sous-étape de tamisage pour récupérer un échantillon très homogène. La granulométrie de l'échantillon après la sous-étape de tamisage est inférieure 500 $\mu$m, en particulier de 5 $\mu$m à 400 $\mu$m, plus particulièrement de 20 $\mu$m à 200 $\mu$m. La sous-étape de tamisage facilite davantage encore l'extraction solide/liquide que la sous-étape de broyage, et permet donc de réduire la durée de l'étape b).

La granulométrie de l'échantillon après la sous-étape de broyage et/ou la sous-étape de tamisage peut être déterminée à l'aide de tamis.

Typiquement, au cours de la sous-étape de pastillage, l'échantillon séché, broyé, voire tamisé, est mélangé avec un liant, le mélange est ensuite introduit dans une capsule de pastillage et la capsule de pastillage est pressée dans une presse telle qu'une presse hydraulique.

Au sens de la présente demande, "liant" désigne tout composé chimique qui permet le maintien de propriétés physiques de la pastille obtenue après le pressage, en particulier qui évite l'effritement de la pastille. A titre de liant, on peut citer l'acide borique, des liants commerciaux comme Boreox, Cereox, Celleox, Multimix, le PVA ou poly(acétate de vinyle), en particulier l'acide borique, le poly(acétate de vinyle), tout particulièrement le liant est l'acide borique.

Le mélange peut par exemple comprendre plus de 25% massique, en particulier de 50% à 90% massique, tout particulièrement de 70% à 80% massique, plus particulièrement 75% d'échantillon séché et broyé, le reste du mélange étant le liant.

La pastille obtenue peut présenter un diamètre de 1 à 10 cm, en particulier de 4 à 9 cm, tout particulièrement de 6 à 8 cm, et plus particulièrement de 7 cm.

La capsule de pastillage peut être pressée à une pression supérieure à 1 tonne, en particulier de 2 à 10 tonnes, tout particulièrement de 3 à 6 tonnes, et plus particulièrement de 5 tonnes.

La sous-étape de pastillage permet d'obtenir une pastille présentant de bonnes propriétés de cohésion. L'utilisation d'un échantillon sous forme de pastille permet d'optimiser la qualité de l'analyse lors de l'étape a) tant quantitativement que qualitativement.

Selon un mode de réalisation, le premier échantillon et le deuxième échantillon sont un seul et unique échantillon. En effet, la spectroscopie par fluorescence aux rayons X est une technique non destructive. Ainsi un premier échantillon ayant subi l'étape a) d'analyse peut ensuite subir l'étape b) d'extraction.

Les inventeurs ont également trouvé que l'indice de réactivité (IR), la concentration massique en sulfates lixiviables et la résistance en compression d'un matériau excavé permettent de conclure de façon fiable, rapidement et en accord avec les recommandations actuelles en quel matériau de construction ce matériau excavé peut être valorisé, par exemple en tant que remblai ou granulat.

Selon un second aspect, la présente invention porte également sur un procédé de sélection d'une filière de valorisation d'un matériau excavé mettant en oeuvre le procédé de détermination tel que défini ci-dessus et comprenant en outre l'étape suivante :
d) mesure de la résistance en compression d'un troisième échantillon de matériau excavé.

Comme expliqué ci-dessus, le procédé de sélection selon l'invention permet avantageusement de déterminer en quelques minutes, de façon fiable, et en accord avec les recommandations actuelles en quel matériau de construction un matériau excavé peut être valorisé.

Selon un mode de réalisation, le procédé de sélection peut comprendre les étapes suivantes :

- détermination d'un indice de réactivité (IR) du matériau excavé et comparaison de cet indice de réactivité (IR) avec une première valeur maximale,
- comparaison de la concentration massique en sulfates lixiviables ($SO_4{}^{2-}{}_{soluble}$) du matériau excavé avec une seconde valeur maximale,
- comparaison de la résistance en compression du matériaux excavé avec une valeur seuil, et

- sélection de la filière de valorisation du matériau excavé selon les critères suivants :
- le matériau excavé peut être valorisé en tant que remblai si sa résistance en compression est inférieure à la valeur seuil, et

  - soit son indice de réactivité (IR) est compris entre la première valeur minimale et la première valeur maximale et sa concentration massique en sulfates lixiviables ($SO_4^{2-}{}_{soluble}$) est inférieure ou égale à la seconde valeur maximale,
  - soit sa concentration massique en sulfates lixiviables ($SO_4^{2-}{}_{soluble}$) est comprise entre la seconde valeur minimale et la seconde valeur maximale et son indice de réactivité (IR) est inférieur ou égal à la première valeur maximale ;

- le matériau excavé peut être valorisé en tant que granulat si :

  sa résistance en compression est supérieure à la valeur seuil, et
  son indice de réactivité (IR) est inférieur ou égal à la première valeur minimale et sa concentration massique en sulfates lixiviables ($SO_4^{2-}{}_{soluble}$) est inférieure ou égale à la seconde valeur maximale.

Au sens de la présente demande, "remblai" désigne un matériau de construction destiné à élever un terrain, combler un creux ou combler les vides de l'exploitation minière et comprenant un matériau excavé valorisable présentant une résistance en compression inférieure à une valeur seuil, et

- soit un indice de réactivité (IR) compris entre une première valeur minimale et la première valeur maximale et une concentration massique en sulfates lixiviables ($SO_4^{2-}{}_{soluble}$) inférieure ou égale à la seconde valeur maximale,
- soit une concentration massique en sulfates lixiviables ($SO_4^{2-}{}_{soluble}$) comprise entre une seconde valeur minimale et la seconde valeur maximale et un indice de réactivité (IR) inférieur ou égal à la première valeur maximale.

Au sens de la présente demande, "granulat" désigne un matériau de construction utilisé pour la réalisation d'ouvrages de Génie Civil, de travaux routiers et de bâtiments et comprenant un matériau excavé valorisable présentant une résistance en compression supérieure à la valeur seuil et un indice de réactivité (IR) inférieur ou égal à une première valeur minimale et une concentration massique en sulfates lixiviables ($SO_4^{2-}{}_{soluble}$) inférieure ou égale à la seconde valeur maximale, en particulier inférieure ou égale à une seconde valeur minimale.

Un granulat pour béton et un granulat pour enrobé sont des exemples de granulat.

Au sens de la présente demande, "granulat pour enrobé" désigne un granulat utilisé pour la confection du bitume et comprenant un matériau excavé valorisable présentant une résistance en compression supérieure à la valeur seuil et un indice de réactivité (IR) inférieur ou égal à la première valeur minimale et une concentration massique en sulfates lixiviables ($SO_4^{2-}{}_{soluble}$) comprise entre la seconde valeur minimale et la seconde valeur maximale.

Au sens de la présente demande, "granulat pour béton" désigne un granulat utilisé pour la confection des bétons et comprenant un matériau excavé présentant une résistance en compression supérieure à la valeur seuil et un indice de réactivité (IR) inférieur ou égal à la première valeur minimale et une concentration massique en sulfates lixiviables ($SO_4^{2-}{}_{soluble}$) inférieure ou égale à la seconde valeur minimale.

La première valeur maximale et la seconde valeur maximale sont telles que définies ci-dessus en lien avec le procédé de détermination de l'invention.

La première valeur minimale peut par exemple être comprise entre 9 et 11, en particulier entre 9,5 et 10,5, tout particulièrement de 10.

Par exemple, la seconde valeur minimale peut être comprise entre 1350 ppm et 1650 ppm, en particulier entre 1425 ppm et 1575 ppm, tout particulièrement entre 1475 ppm et 1525 ppm.

La valeur seuil peut par exemple être comprise entre 45 MPa et 55 MPa, en particulier entre 47 MPa et 52 MPa, plus particulièrement être de 50 MPa.

De façon avantageuse, la conclusion sur le choix de filière de valorisation d'un matériau excavé est fiable, rapide et en accord avec les recommandations actuelles lorsque la première valeur minimale, la seconde valeur minimale et la valeur seuil sont dans les plages décrites ci-dessus.

L'étape d) de mesure de la résistance en compression peut être réalisée par des techniques connues de l'homme du métier, par exemple les techniques décrites dans la norme NF EN 16907-2 de décembre 2018 ou la norme NF EN 1926 d'avril 2007.

Selon un mode de réalisation, le premier échantillon et le troisième échantillon sont un seul et unique échantillon. En effet, la spectroscopie par fluorescence aux rayons X est une technique non destructive. Ainsi un premier échantillon ayant subi l'étape a) d'analyse peut ensuite subir l'étape d) de mesure.

Selon un mode de réalisation, le premier échantillon, le deuxième échantillon et le troisième échantillon sont un seul et unique échantillon. En effet, la spectroscopie par fluorescence aux rayons X est une technique non destructive. Ainsi un premier échantillon ayant subi l'étape a) d'analyse peut ensuite subir l'étape d) de mesure puis ensuite subie l'étape b) d'extraction.

Selon un troisième aspect, la présente invention porte également sur un procédé de production d'un matériau de construction comprenant une ou des étapes de mise en forme d'un matériau excavé dont un ou des échantillons ont subi les étapes a) à c) du procédé de détermination tel que défini ci-dessus et/ou l'étape d) du procédé de sélection tel que défini ci-dessus.

Les étapes de mise en forme du procédé de production selon l'invention sont des étapes classiques que l'homme du métier saura adapter au matériau excavé valorisable pour produire un produit de construction.

Ainsi, selon un mode de réalisation du procédé de production de l'invention, le matériau de construction peut être un remblai ou un granulat, en particulier un granulat pour béton ou un granulat pour enrobé.

Le procédé de production de l'invention permet de produire un matériau de construction. Ainsi l'invention porte également sur un matériau de construction comprenant un matériau excavé valorisable présentant un indice de réactivité (IR) inférieur ou égal à une première valeur maximale et une concentration massique en sulfates lixiviables inférieure ou égale à une seconde valeur maximale,

l'indice de réactivité (IR) étant déterminé selon l'équation suivante:

$$IR = 100 * \frac{0{,}33 * SO_{4\ \text{soluble}}^{2-} + Cl_{\text{soluble}}^{-}}{S_{\text{total}} + Cl_{\text{total}}}$$

avec $SO_{4\ \text{soluble}}^{2-}$ la concentration massique en sulfates lixiviables du matériau excavé, $Cl_{\text{soluble}}^{-}$ la concentration massique en chlorures lixiviables du matériau excavé, $S_{\text{total}}$ la concentration massique en soufre total du matériau excavé et $Cl_{\text{total}}$ la concentration massique en chlore total du matériau excavé.

La première valeur maximale et la seconde valeur maximale sont telles que définies ci-dessus en lien avec le procédé de détermination de l'invention.

Selon un mode de réalisation, le matériau de construction peut être un remblai ou un granulat, en particulier un granulat pour béton ou un granulat pour enrobé.

Le remblai, le granulat, le granulat pour béton et le granulat pour enrobé sont tels que définis ci-dessus en lien avec le procédé de production de l'invention.

Un exemple, qui ne forme pas partie de la présente invention, porte également sur un laboratoire mobile comprenant des équipements de laboratoire adaptés à la mise en oeuvre d'au moins une des étapes a), b) et c) du procédé de détermination tel que défini ci-dessus et/ou des équipements de laboratoire adaptés à la mise en oeuvre de l'étape d) du procédé de sélection tel que défini ci-dessus.

En effet, l'analyse par spectroscopie par fluorescence aux rayons X peut être mise en oeuvre à l'aide des équipements portables de laboratoire. L'étape b) d'extraction peut être réalisée dans un réacteur de petit volume. Les équipements de laboratoire adaptés à la mise en oeuvre des analyses de l'étape c), en particulier le dosage potentiométrique et le dosage conductimétrique, et les équipements de laboratoire permettant de mesurer la résistance en compression lors de l'étape d) ne sont pas encombrants. Ces étapes peuvent donc être mises en oeuvre dans le laboratoire mobile selon l'invention qui peut, par conséquent, être positionné directement sur le site d'excavation des matériaux à analyser. Cela permet d'éviter tout transfert des matériaux d'excavation vers un laboratoire extérieur au site d'excavation, réduisant ainsi fortement les coûts logistiques.

Ces équipements de laboratoire sont également standards. L'homme du métier saura les choisir en fonction des étapes des procédés de détermination et de sélection selon l'invention qu'il souhaite réaliser.

L'invention va être décrite plus en détail à l'aide des exemples suivants donnés à titre d'illustration seulement.

## Exemples

Exemple 1 : Analyse d'un premier matériau excavé selon la méthode de détermination selon l'invention

Une masse connue (30 g) d'un échantillon de matériau excavé issu du chantier du creusement du tunnel Lyon-Turin sur l'attaque de la descenderie de Saint Martin de la Porte est séchée dans une étuve à 145°C pendant 30 minutes pour déterminer la matière sèche dudit échantillon.

10 g de cet échantillon séché sont broyés dans un broyeur en oxyde de zirconium pendant 1 min, récupérés puis tamisé à 200 μm.

La concentration massique en chlore et en soufre contenus dans cet échantillon tamisé sont analysées par spectroscopie par fluorescence aux rayons X selon la procédure de la norme NF EN 12457-2.

On ajoute une masse de l'échantillon séché équivalente à 4 g de matière sèche de cet échantillon dans un cylindre Q-Cup de l'appareil Extraction Dispersive Énergisée Guidée (EDGE) de CEM. Le cylindre Q-Cup est équipé d'un filtre Q-Disc C9 en cellulose hydrophile et dont la porosité est de 1 μm/55μm. L'échantillon est ensuite chauffé à la température T1 de 60°C puis le solvant, qui est de l'eau, est introduit dans le cylindre Q-cup de part et d'autre de l'échantillon. Le mélange est ensuite chauffé à la température $T_{\text{extraction}}$ de 100°C. Cette température $T_{\text{extraction}}$ est maintenue pendant 30 secondes. L'extraction dure donc 30 secondes.

Après les 30 secondes, le mélange n'est plus chauffé de sorte que la température du mélange diminue. Le lixiviat est ensuite séparé de la fraction solide à travers le filtre Q-Disc par application d'une surpression de 2,2 bar dans le cylindre Q-Cup. Le lixiviat est ensuite séparé en deux lots de 40 mL. Le premier lot permet de déterminer la concentration massique en chlorure lixiviables par dosage potentiométrique du lixiviat en plongeant une électrode sélective de chlorure à membrane cristalline et une électrode de référence Ag/AgCl dans le lixiviat. Le second lot permet déterminer la concentration massique en sulfates lixiviables par dosage conductimétrique à l'aide d'une solution titrante de BaCl$_2$ à 0,05 mol/L.

Le Tableau 1 ci-dessous récapitule les concentrations massiques obtenues par le procédé de détermination de l'invention.

[Tableau 1]

| Concentration massique dans le matériau excavé de l'Exemple 1 | |
| --- | --- |
| Soufre total | 4791 ppm |
| Clore total | < Limite de détection |
| SO$_4^{2-}$ lixiviable | < Limite de détection |
| Cl$^-$ lixiviable | < Limite de détection |

L'indice de réactivité de ce matériau excavé est de 0, il est donc inférieur à la première valeur minimale. La concentration en SO$_4^{2-}$ lixiviable de ce matériau excavé n'est pas détectable, elle est donc inférieure à la seconde valeur minimale. La résistance en compression de ce matériau mesurée selon la norme NF EN 1926 d'avril 2007 est supérieure à 50 MPa.

Ce matériau excavé peut être valorisé en tant que granulat pour béton.

L'analyse de ce matériau excavé avec le procédé selon l'invention a duré 120 minutes. La même analyse réalisée selon les normes actuellement en vigueur aurait duré au moins 5 jours.

De plus le procédé selon l'invention permet de choisir la même filière de valorisation que les recommandations actuelles.

Exemple 2 : Analyse d'un second matériau excavé selon la méthode de détermination selon l'invention

Un échantillon de matériau excavé issu du même chantier que l'Exemple 1 et subit les mêmes étapes que celle décrite dans l'Exemple 1.

Le Tableau 2 ci-dessous récapitule les concentrations massiques obtenues par le procédé de détermination de l'invention.

[Tableau 2]

| Concentration massique dans le matériau excavé de l'Exemple 2 | |
| --- | --- |
| Soufre total | 14415 ppm |
| Clore total | < Limite de détection |
| SO$_4^{2-}$ lixiviable | 1537 ppm |
| Cl$^-$ lixiviable | < Limite de détection |

L'indice de réactivité de ce matériau excavé est de 3,5, il est donc inférieur à la première valeur minimale.

La concentration en SO$_4^{2-}$ lixiviable de ce matériau excavé est de 1537 ppm, *i.e.* comprise entre la seconde valeur minimale et la seconde valeur maximale. La résistance en compression de ce matériau mesurée selon la norme NF EN 1926 d'avril 2007 est inférieure à 50 MPa.

Ce matériau excavé peut être valorisé en tant que remblai.

L'analyse de ce matériau excavé avec le procédé selon l'invention a duré 120 minutes. La même analyse réalisée selon les normes actuellement en vigueur aurait duré au moins 5 jours.

De plus le procédé selon l'invention permet de choisir la même filière de valorisation que les recommandations actuelles.

Exemple 3 : Analyse d'un second matériau excavé selon la méthode de détermination selon l'invention

Un échantillon de matériau excavé issu du même chantier que l'Exemple 1 subit les mêmes étapes que celle décrite dans l'Exemple 1.

Le Tableau 3 ci-dessous récapitule les concentrations massiques obtenues par le procédé de détermination de l'invention.

[Tableau 3]

| Concentration massique dans le matériau excavé de l'Exemple 3 | |
| --- | --- |
| Soufre total | 184 912 ppm |
| Clore total | < Limite de détection |
| SO$_4^{2-}$ lixiviable | 15 300 ppm |
| Cl$^-$ lixiviable | < Limite de détection |

La concentration en SO$_4^{2-}$ lixiviable de ce matériau excavé est de 15 300 ppm, *i.e.* est supérieure à la seconde valeur maximale. Ce matériau excavé ne peut pas être valorisé même si son indice de réactivité de 2,7 est inférieur à la première valeur minimale.

L'analyse de ce matériau excavé et le choix de la filière de revalorisation avec le procédé selon l'invention à durée 120 minutes et est en accord avec les recommandations actuelles.

**Revendications**

1. Procédé de détermination du caractère valorisable d'un matériau excavé comprenant les étapes suivantes:

   a) analyse d'activation par neutrons gamma rapides, analyse par spectroscopie par fluorescence aux rayons X ou leur combinaison d'un premier échantillon du matériau excavé pour déterminer la concentration massique en soufre ($S_{total}$) et la concentration massique en chlore ($Cl_{total}$) du matériau excavé,

b) extraction solide/liquide à une température $T_{extraction}$ de 65°C à 200°C d'un second échantillon du matériau excavé avec un solvant liquide pour obtenir un mélange comprenant un lixiviat et une fraction solide, et

c) analyse chimique, analyse physique ou leur combinaison dudit lixiviat pour déterminer la concentration massique en sulfates lixiviables ($SO_4^{2-}{}_{soluble}$) et la concentration massique en chlorures lixiviables ($Cl^-{}_{soluble}$) du matériau excavé.

2. Procédé selon la revendication 1 dans lequel l'étape a) d'analyse par spectroscopie par fluorescence aux rayons X est réalisée avec une énergie variant de 0 à 100 keV.

3. Procédé selon la revendication 1 ou la revendication 2, dans lequel le temps total d'acquisition lors de l'étape a) d'analyse par spectroscopie par fluorescence aux rayons X est de 1 min à 120 min.

4. Procédé selon la revendication 1 dans laquelle le solvant liquide de l'étape b) d'extraction est choisi parmi l'eau, une solution aqueuse, un solvant organique, un solvant inorganique et leurs mélanges.

5. Procédé selon la revendication 1 ou la revendication 4 dans lequel le ratio entre volume de solvant liquide et la masse sèche du second échantillon lors de l'étape b) d'extraction est de 5 ml/g à 20 ml/g.

6. Procédé selon l'une quelconques des revendications 1, 4 et 5 dans lequel l'étape b) est réalisée à une pression $P_{extraction}$ de 1 bar à 10 bar.

7. Procédé selon l'une quelconques des revendications 1, 4 à 6 dans lequel l'étape b) d'extraction est réalisée dans un réacteur comprenant :

- une chambre d'extraction adaptée pour recevoir le second échantillon du matériau excavé, et
- deux ports d'entrée de liquide et un port de sortie de liquide,

dans lequel

les deux ports d'entrée de liquide sont fluidiquement connectés à la chambre d'extraction et positionnés de part et d'autre de la chambre d'extraction, et
le port de sortie de liquide est fluidiquement connecté à la chambre d'extraction.

8. Procédé selon l'une quelconque des revendications 1 à 7 dans lequel l'analyse chimique de l'étape c) d'analyse est choisie parmi un dosage conductimétrique, un dosage par chromatographie ionique en phase liquide, un dosage colorimétrique, un dosage potentiométrique, un dosage pH-métrique et leurs combinaisons, et l'analyse physique de l'étape c) d'analyse est choisie parmi une spectrométrie d'absorption, une spectrométrie d'émission atomique avec plasma à couplage inductif (ICP-AES), une spectrométrie de masse avec plasma à couplage inductif (ICP-MS), une spectrométrie d'ionisation de flamme (FID), une spectrométrie d'émission de flamme, une spectrophotométrie UV, et leurs combinaisons, en particulier une spectrométrie de masse avec plasma à couplage inductif (ICP-MS).

9. Procédé selon l'une quelconques des revendications 1 à 8 comprenant entre l'étape b) et l'étape c) une étape de séparation du mélange comprenant le lixiviat et la fraction solide pour obtenir le lixiviat, cette étape de séparation étant réalisée à l'aide d'un filtre dont le matériau est choisi parmi une membrane de filtration, de la fibre de verre, de la cellulose, le PTFE, du nylon, du PMMA, du PE, des matériaux sulfonés, des matériaux acryliques, des matériaux fluorés.

10. Procédé de sélection d'une filière de valorisation d'un matériau excavé mettant en oeuvre le procédé de détermination tel que défini dans l'une quelconque des revendications 1 à 9 et comprenant en outre l'étape suivante :

d) mesure de la résistance en compression d'un troisième échantillon de matériau excavé.

11. Procédé de production d'un matériau de construction comprenant une ou des étapes de mise en forme d'un matériau excavé dont un ou des échantillons ont subi les étapes a) à c) du procédé de détermination tel que défini dans l'une quelconque des revendications 1 à 9 et l'étape d) du procédé de sélection tel que défini dans la revendication 10.

**Patentansprüche**

1. Verfahren zur Bestimmung der Verwertbarkeit von ausgehobenem Material, das die folgenden Schritte umfasst:

a) Aktivierungsanalyse mit schnellen Gammaneutronen, Analyse mit Röntgenfluoreszenzspektroskopie oder deren Kombination einer ersten Probe des ausgehobenen Materials, um die Massenkonzentration von Schwefel ($S_{total}$) und die Massenkonzentration von Chlor ($Cl_{total}$) des ausgehobenen Materials zu bestimmen,

b) Fest/Flüssig-Extraktion bei einer Temperatur

$T_{extraction}$ von 65 °C bis 200 °C einer zweiten Probe des ausgehobenen Materials mit einem flüssigen Lösungsmittel, um eine Mischung zu erhalten, die ein Sickerwasser und eine feste Fraktion umfasst, und

c) chemische Analyse, physikalische Analyse oder deren Kombination des Sickerwassers, um die Massenkonzentration an auslaugbaren Sulfaten ($SO_4^{2-}_{soluble}$) und die Massenkonzentration an auslaugbaren Chloriden ($Cl^-_{soluble}$) des ausgehobenen Materials zu bestimmen.

2. Verfahren nach Anspruch 1, wobei Schritt a) der Analyse durch Röntgenfluoreszenzspektroskopie mit einer Energie im Bereich von 0 bis 100 keV durchgeführt wird.

3. Verfahren nach Anspruch 1 oder 2, wobei die gesamte Erfassungszeit in Schritt a) der Analyse durch Röntgenfluoreszenzspektroskopie 1 min bis 120 min beträgt.

4. Verfahren nach Anspruch 1, wobei das flüssige Lösungsmittel in Extraktionsschritt b) gewählt ist aus Wasser, einer wässrigen Lösung, einem organischen Lösungsmittel, einem anorganischen Lösungsmittel und deren Mischungen.

5. Verfahren nach Anspruch 1 oder Anspruch 4, wobei das Verhältnis zwischen dem Volumen des flüssigen Lösungsmittels und der Trockenmasse der zweiten Probe in Extraktionsschritt b) 5 ml/g bis 20 ml/g beträgt.

6. Verfahren nach einem der Ansprüche 1, 4 oder 5, wobei Schritt b) bei einem Druck $P_{extraction}$ von 1 bar bis 10 bar durchgeführt wird.

7. Verfahren nach einem beliebigen der Ansprüche 1, 4 bis 6, wobei Schritt b) der Extraktion in einem Reaktor durchgeführt wird, umfassend:

- eine Extraktionskammer, die dazu ausgebildet ist, die zweite Probe des ausgehobenen Materials aufzunehmen, und
- zwei Flüssigkeitseinlassports und einen Flüssigkeitsauslassport,

wobei
die beiden Flüssigkeitseinlassports fluidisch mit der Extraktionskammer verbunden und auf beiden Seiten der Extraktionskammer positioniert sind, und der Flüssigkeitsauslassport fluidisch mit der Extraktionskammer verbunden ist.

8. Verfahren nach einem der Ansprüche 1 bis 7, wobei die chemische Analyse in Analyseschritt c) gewählt ist aus konduktometrischer Bestimmung, flüssig-keitschromatographischer Ionenbestimmung, kolorimetrischer Bestimmung, potentiometrischer Bestimmung, pH-metrischer Bestimmung und deren Kombinationen, und die physikalische Analyse in Analyseschritt c) gewählt ist aus Absorptionsspektrometrie, Atomemissionsspektrometrie mit induktiv gekoppeltem Plasma (ICP-AES), Massenspektrometrie mit induktiv gekoppeltem Plasma (ICP-MS), Flammenionisationsspektrometrie (FID), Flammenemissionsspektrometrie, UV-Spektrophotometrie und deren Kombinationen, insbesondere Massenspektrometrie mit induktiv gekoppeltem Plasma (ICP-MS).

9. Verfahren nach einem der Ansprüche 1 bis 8, umfassend zwischen Schritt b) und Schritt c) einen Schritt der Trennung der Mischung, die das Sickerwasser und die feste Fraktion umfasst, um das Sickerwasser zu erhalten, wobei dieser Trennschritt mit Hilfe eines Filters durchgeführt wird, dessen Material aus einer Filtermembran, Glasfaser, Zellulose, PTFE, Nylon, PMMA, PE, sulfonierten Materialien, Acrylmaterialien, fluorierten Materialien gewählt ist.

10. Verfahren zur Auswahl eines Verwertungsweges für ein ausgehobenes Material, bei dem das Bestimmungsverfahren, wie in einem der Ansprüche 1 bis 9 definiert, durchgeführt wird und welches ferner den folgenden Schritt umfasst: d) Messen der Druckfestigkeit einer dritten Probe des ausgehobenen Materials.

11. Verfahren zur Herstellung eines Baumaterials, umfassend einen oder mehrere Schritte des Formens eines ausgehobenen Materials, von dem eine oder mehrere Proben die Schritte a) bis c) des Bestimmungsverfahrens, wie in einem der Ansprüche 1 bis 9 definiert, und den Schritt d) des Auswahlverfahrens, wie in Anspruch 10 definiert, durchlaufen haben.

## Claims

1. A method for determining the usable nature of an excavated material comprising the following steps:

a) analysis of activation by fast gamma neutrons, X-ray fluorescence spectroscopy analysis or the combination thereof of a first sample of the excavated material to determine the mass concentration of sulphur ($S_{total}$) and the mass concentration of chlorine ($Cl_{total}$) of the excavated material,

b) solid/liquid extraction at a temperature $T_{extraction}$ of 65°C to 200°C of a second sample of the excavated material with a liquid solvent to obtain a mixture comprising a leachate and a solid fraction, and

c) chemical analysis, physical analysis or the combination thereof of said leachate to determine the mass concentration of leachable sulphates ($SO_4^{2-}{}_{soluble}$) and the mass concentration of leachable chlorides ($Cl^-{}_{soluble}$) of the excavated material.

2. The method according to claim 1, wherein step a) of X-ray fluorescence spectroscopy analysis is carried out with an energy ranging from 0 to 100 keV.

3. The method according to claim 1 or claim 2, wherein the total acquisition time during step a) of X-ray fluorescence spectroscopy analysis is 1 min to 120 min.

4. The method according to claim 1, wherein the liquid solvent of the extraction step b) is selected from water, an aqueous solution, an organic solvent, an inorganic solvent and the mixtures thereof.

5. The method according to claim 1 or claim 4, wherein the ratio between volume of liquid solvent and the dry mass of the second sample during the extraction step b) is 5 ml/g to 20 ml/g.

6. The method according to any one of claims 1, 4 and 5, wherein step b) is carried out at a pressure $P_{extraction}$ of 1 bar to 10 bar.

7. The method according to any one of claims 1, 4 to 6, wherein the extraction step b) is carried out in a reactor comprising:

   - an extraction chamber adapted to receive the second sample of the excavated material, and
   - two liquid inlet ports and one liquid outlet port,

   Wherein

   the two liquid inlet ports are fluidly connected to the extraction chamber and positioned on either side of the extraction chamber, and
   the liquid outlet port is fluidly connected to the extraction chamber.

8. The method according to any one of claims 1 to 7, wherein the chemical analysis of the analysis step c) is selected from a conductimetric assay, an ion chromatography assay in the liquid phase, a colorimetric assay, a potentiometric assay, a pH-metric assay and combinations thereof, and the physical analysis of the analysis step c) is selected from an absorption spectrometry, an inductively coupled plasma atomic emission spectrometry (ICP-AES), an inductively coupled plasma mass spectrometry (ICP-MS), a flame ionization spectrometry (FID), a flame emission spectrometry, a UV spectrophotometry, and combinations thereof, in particular an inductively coupled plasma mass spectrometry (ICP-MS).

9. The method according to any one of claims 1 to 8, comprising, between step b) and step c), a step of separating the mixture comprising the leachate and the solid fraction to obtain the leachate, this separation step being carried out using a filter whose material is selected from a filtration membrane, fiberglass, cellulose, PTFE, nylon, PMMA, PE, sulphonated materials, acrylic materials, fluorinated materials.

10. The method for selecting a sector for using an excavated material implementing the determination method as defined in any one of claims 1 to 9, and further comprising the following step:

    d) measuring the compressive strength of a third sample of excavated material.

11. The method for producing a construction material comprising one or more steps of shaping an excavated material, of which one or more samples have undergone steps a) to c) of the determination method as defined in any one of claims 1 to 9 and step d) of the selection method as defined in claim 10.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **MATSUMOTO SHINJI et al.** Characterization of Mine Waste and Acid Mine Drainage Prédiction by Simple Testing Methods in Terms of the Effects of Sulfate-Sulfur and Carbonate Minerals. *MINERALS,* 01 Septembre 2018, vol. 8 (9